# EUROPEAN PATENT APPLICATION

(11) **EP 1 170 017 A1**
(43) Date of publication of application: **09.01.2002**
(21) Application number: 01117491.9
(22) Date of filing: 12.05.1998
(51) Int. Cl.: A61K 39/395, A61K 31/00, A61K 31/505, A61K 38/17, A61P 37/00

(54) **Use of tumor necrosis factor alpha (TNF-alpha) and vascular endothelial growth factor (VEGF) for the manufacture of a therapeutic composition**

(30) Priority: 12.05.1997 US 854881
(62) Divisional of application: 98920669.3
(71) Applicant: THE KENNEDY INSTITUTE OF RHEUMATOLOGY, Hammersmith, London W6 8LH (GB)
(72) Inventor: Feldmann, Marc, Highgate, London N6 4QT (GB); Maini, Ravinder Nath, Barnes, London SW13 9EW (GB); Paleolog, Ewa Maria, Ealing, London W5 3JR (GB)
(74) Representative: Kirkham, Nicholas Andrew

(57) **Abstract**

Methods for treating and/or preventing a TNF-mediated disease in an individual are disclosed. Also disclosed are compositions comprising a TNFα antagonist and a VEGF antagonist. TNF-mediated diseases include rheumatoid arthritis, Crohn's disease, and acute and chronic immune diseases associated with transplantation.

## Description

### BACKGROUND OF THE INVENTION

Monocytes and macrophages secrete cytokines known as tumor necrosis factor alpha (TNFα), interleukin-1 (IL-1) and interleukin-6 (IL-6) in response to endotoxin or other stimuli. TNFα is a soluble homotrimer of 17 kD protein subunits (Smith *et al., J. Biol. Chem., 262*:6951-6954 (1987)). A membrane-bound 26 kD precursor form of TNF also exists (Kriegler *et al., Cell, 53*:45-53 (1988)). For reviews of TNF, see Beutler *et al., Nature, 320*:584 (1986); Old, *Science, 230*:630 (1986); and Le *et al., Lab. Invest., 56*:234 (1987).

Cells other than monocytes or macrophages also produce TNFα. For example, human non-monocytic tumor cell lines produce TNFα (Rubin *et al., J. Exp. Med., 164*:1350 (1986); Spriggs *et al., Proc. Natl. Acad. Sci. USA, 84*:6563 (1987)). CD4+ and CD8+ peripheral blood T lymphocytes and some cultured T and B cell lines (Cuturi *et al., J. Exp. Med., 165*:1581 (1987); Sung *et al., J. Exp. Med., 168:1539* (1988); Turner *et al., Eur. J. Immunol., 17*:1807-1814 (1987)) also produce TNFα.

TNFα causes pro-inflammatory actions which result in tissue injury, such as degradation of cartilage and bone (Saklatvala, *Nature, 322*:547-549 (1986); Bertolini, *Nature, 319*:516-518 (1986)), induction of adhesion molecules, inducing procoagulant activity on vascular endothelial cells (Pober *et al., J. Immunol., 136*:1680 (1986)), increasing the adherence of neutrophils and lymphocytes (Pober *et al*., *J*. *Immunol., 138*:3319 (1987)), and stimulating the release of platelet activating factor from macrophages, neutrophils and vascular endothelial cells (Camussi *et al., J. Exp. Med., 166*:1390 (1987)).

There is evidence that associates TNFα with infections (Cerami *et al., Immunol. Today, 9*:28 (1988)), immune disorders, neoplastic pathologies (Oliff *et al., Cell, 50*:555 (1987)), autoimmune pathologies and graft-versus-host pathologies (Piguet *et al., J*. *Exp. Med., 166*:1280 (1987)). The association of TNFα with cancer and infectious, pathologies is often related to the host's catabolic state. Cancer patients suffer from weight loss, usually associated with anorexia.

The extensive wasting which is associated with cancer, and other diseases, is known as "cachexia" (Kern *et al., J. Parent. Enter. Nutr., 12*:286-298 (1988)). Cachexia includes progressive weight loss, anorexia, and persistent erosion of lean body mass in response to a malignant growth. The cachectic state causes much cancer morbidity and mortality. There is evidence that TNFα is involved in cachexia in cancer, infectious pathology, and other catabolic states (see, e.g., Beutler and Cerami, *Ann. Rev. Immunol., 7*:625-655 (1989)).

TNFα is believed to play a central role in gram-negative sepsis and endotoxic shock (Michie *et al., Br. J. Surg., 76*:670-671 (1989); Debets *et al., Second Vienna Shock Forum,* p. 463-466 (1989); *Simpson et al., Crit. Care Clin., 5*:27-47 (1989)), including fever, malaise, anorexia, and cachexia. Endotoxin strongly activates monocyte/macrophage production and secretion of TNFα and other cytokines (Kornbluth *et al., J. Immunol., 137*:2585-2591 (1986)). TNFα and other monocyte-derived cytokines mediate the metabolic and neurohormonal responses to endotoxin (Michie *et al., New Engl. J. Med., 318*:1481-1486 (1988)). Endotoxin administration to human volunteers produces acute illness with flu-like symptoms including fever, tachycardia, increased metabolic rate and stress hormone release (Revhaug *et al., Arch. Surg., 123*:162-170 (1988)). Circulating TNFα increases in patients suffering from Gram-negative sepsis (Waage *et al., Lancet, 1*:355-357 (1987); Hammerle *et al., Second Vienna Shock Forum,* p. 715-718 (1989); Debets *et al., Crit. Care Med., 17*:489-497 (1989); Calandra *et al., J. Infect. Dis., 161*:982-987 (1990)).

Thus, TNFα has been implicated in inflammatory diseases, autoimmune diseases, viral, bacterial and parasitic infections, malignancies, and/or neurogenerative diseases and is a useful target for specific biological therapy in diseases, such as rheumatoid arthritis and Crohn's disease. Beneficial effects in open-label trials with a chimeric monoclonal antibody to TNFα (cA2) have been reported with suppression of inflammation and with successful retreatment after relapse in rheumatoid arthritis (Elliott *et al., Arthritis Rheum., 36*:1681-1690 (1993); and Elliott *et al., Lancet, 344*:1125-1127 (1994)) and in Crohn's disease (Van Dullemen *et al., Gastroenterology, 109*:129-135 (1995)). Beneficial results in a randomized, double-blind, placebo-controlled trial with cA2 have also been reported in rheumatoid arthritis with suppression of inflammation (Elliott *et al., Lancet, 344*:1105-1110 (1994)). These beneficial effects are mediated, in part, by reduced trafficking of inflammatory cells to the synovium and by suppression of the release of pro-inflammatory cytokines such as IL-1 (Brennan *et al., Lancet, 2*:244-247 (1989); Feldmann *et al., Ann. Rev. Immunol., 14*:397-440 (1996); and Paleolog *et al., Arthritis Rheum., 39*:1082-1091 (1996)).

Vascular endothelial growth factor (VEGF), also known as vascular permeability factor or vasculotropin, is a diffusible endothelial cell-specific mitogen and angiogenic factor that can also increase vascular permeability (Ferrara, *Breast Cancer Res. Treat., 36*(2):127-137 (1995)). VEGF is a disulfide-linked homodimeric glycoprotein of about 34-45 kDa consisting of four isoforms (containing either 121, 165, 189 or 206 amino acid residues in the mature monomer (see, e.g., Ferrara, *Breast Cancer Res. Treat., 36*:127-137 (1995); Dvorak *et al., Am. J. Path., 146*(5):1029-1039 (1995); and Thomas, *J. Biol. Chem., 271:*603-606 (1996)). VEGF₁₂₁ and to a large extent VEGF₁₆₅ are secreted in soluble form, whereas VEGF₁₈₉ and VEGF₂₀₆ remain cell-associated (see, e.g., Ferrara, *Breast Cancer Res. Treat., 36*:127-137 (1995); Dvorak *et al., Am. J. Path., 146*(5):1029-1039 (1995) ; and Thomas, *J. Biol. Chem., 271*:603-606 (1996)).

VEGF stimulates endothelial cell growth and increases microvascular permeability by interacting with membrane-spanning tyrosine kinase receptors, the fms-like tyrosine kinase receptor (Flt) (deVries *et al., Science, 255*:989-991 (1992); and Shibuya *et al., Oncogene, 5*:519-524 (1990)) and kinase insert domain-containing receptor (KDR) (Terman *et al., Biochem. Biophys. Res. Commun., 187*:1579-1586 (1992)). The murine homolog of KDR is fetal liver kinase receptor (Flk) (Quinn *et al.*, *Proc. Natl. Acad. Sci. USA, 90*:7533-7537 (1993)). (See also, *Ferrara, Breast Cancer Res. Treat., 36*:127-137 (1995); and Dvorak *et al., Am. J. Path., 146*(5):1029-1039 (1995)).

Recent evidence associates VEGF with the pathogenesis of a range of diseases associated with angiogenesis. For example, VEGF has been implicated in chronic vascular hyperpermeability and angiogenesis of solid and ascites tumors, healing wounds, rheumatoid arthritis, psoriasis and diabetic retinopathy (see, e.g., Brown *et al., J. Immunol., 154*(6):2801-2807 (1995); Dvorak *et al., Int. Arch. Allergy Immunol., 107*:233-235 (1995); Ferrara, *Breast Cancer Res. Treat., 36*(2):127-137 (1995); Aiello *et al., Proc. Natl. Acad. Sci. USA, 92*(23):10457-10461 (1995); Adamis *et al., Arch. Ophthalmol., 114*:66-71 (1996); Koch *et al., J.* *Immunol., 152*:4149-4156 (1994); and Peacock *et al., J. Exp. Med., 175*:1135-1138 (1992)).

VEGF expression has been reported to be elevated in pathological conditions including cancer, proliferative retinopathy, psoriasis and rheumatoid arthritis (RA) (see, e.g., Claffey *et al., Cancer Metastasis Rev., 15*(2):165-176 (1996) ; and Koch *et al., J. Immunol., 152*:4149-4156 (1994)). For example, serum VEGF levels have been reported to be higher in individuals with POEMS (polyneuropathy, organomegaly, endocrinopathy, M-protein and skin changes) syndrome than in normal individuals (Kondo *et al., Biochim. Biophys. Acta, 1221*:211-214 (1994); Soubrier *et al., Arth. Rheum., 39*:S131 (1996); and Watanabe *et al., Lancet, 347*:702 (1996)).

VEGF protein and mRNA have been localised to macrophages and lining cells in synovial membranes from RA patients, and VEGF receptors are expressed by RA synovial endothelial cells, the putative target of VEGF (Koch *et al., J. Immunol, 152*:4149-4156 (1994); and Fava *et al., J. Exp. Med., 180*:341-346 (1994)). Increased production of VEGF in response to chronic hypoxia has been reported (Tuder *et al., J. Clin. Invest., 95*:1798-1807 (1995)), and hypoxic conditions prevail in RA when the elevated intra-articular pressure exceeds synovial capillary pressure (Blake *et al., Lancet, 8633*:289-293 (1989)).

In animal models of RA, inhibitors of angiogenesis have been found to prevent onset of disease and significantly suppress established arthritis, in parallel with inhibition of pannus formation and reduced serum VEGF concentrations (Oliver *et al., Cellular Immunol., 157*:291-299 (1994); and Oliver *et al., Cellular Immunol., 166*:196-206 (1996)).

However, improved methods for treating autoimmune diseases, such as rheumatoid arthritis, are desirable.

### SUMMARY OF THE INVENTION

The present invention provides methods for treating and/or preventing a tumor necrosis factor-mediated disease in an individual in need thereof comprising co-administering a tumor necrosis factor alpha (TNFα) antagonist and a vascular endothelial growth factor (VEGF) antagonist to the individual in therapeutically effective or synergistic amounts. The present invention also provides methods for treating and/or preventing recurrence of a TNF-mediated disease in an individual comprising co-administering a TNFα antagonist and a VEGF antagonist to the individual in therapeutically effective amounts. TNF-mediated diseases include rheumatoid arthritis, Crohn's disease, and acute and chronic immune diseases associated with an allogenic transplantation (e.g., renal, cardiac, bone marrow, liver, pancreatic, small intestine, skin or lung transplantation).

In one embodiment, the invention relates to a method of treating and/or preventing (such as preventing relapse of) rheumatoid arthritis in an individual comprising co-administering a TNFα antagonist and a VEGF antagonist to the individual in therapeutically effective amounts. In a second embodiment, the invention relates to a method of treating and/or preventing (such as preventing relapse of) Crohn's disease in an individual comprising co-administering a TNFα antagonist and a VEGF antagonist to the individual in therapeutically effective amounts. In a third embodiment, the invention relates to a method of treating and/or preventing acute or chronic immune disease associated with a transplantation in an individual comprising co-administering a TNFα antagonist and a VEGF antagonist to the individual in therapeutically effective amounts.

Other therapeutic regimens and agents can be used in combination with the therapeutic co-administration of TNFα antagonists and VEGF antagonists. For example, in a particular embodiment, methotrexate is co-administered with the TNFα antagonist and the VEGF antagonist in therapeutically effective or synergistic amounts.

The invention further relates to compositions comprising a TNFα antagonist and a VEGF antagonist, such as for use in therapy or in the manufacture of a medicament for treating the above diseases. In a particular embodiment, the composition further comprises methotrexate.

TNFα antagonists useful in the invention include anti-TNFα antibodies and antigen-binding fragments thereof; receptor molecules which bind specifically to TNFα; compounds which prevent and/or inhibit TNFα synthesis, TNFα release or its action on target cells, such as thalidomide, tenidap, phosphodiesterase inhibitors (e.g, pentoxifylline and rolipram), A2b adenosine receptor agonists and A2b adenosine receptor 'enhancers; compounds which prevent and/or inhibit TNFα receptor signalling, such as mitogen activated protein (MAP) kinase inhibitors; compounds which block and/or inhibit membrane TNFα cleavage, such as metalloproteinase inhibitors; compounds which block and/or inhibit TNFα activity, such as angiotensin converting enzyme (ACE) inhibitors (e.g., captopril); and compounds which block and/or inhibit TNFα production and/or synthesis, such as MAP kinase inhibitors.

VEGF antagonists useful in the invention include anti-VEGF antibodies and antigen-binding fragments thereof; receptor molecules which bind specifically to VEGF; compounds which prevent and/or inhibit VEGF function (e.g., suramin, protein tyrosine kinase (PTK) inhibitors (e.g., lavendustin A); compounds which prevent and/or inhibit binding of VEGF to VEGF receptors or extracellular domains thereof (e.g., platelet factor-4 (PF-4)); compounds which block and/or interfere with VEGF receptor signalling; and compounds which block and/or interfere with VEGF activation (e.g., mithramycin). VEGF antagonists useful in the invention also include agents which are antagonists of signals that drive VEGF production and/or synthesis, such as agents which decrease and/or block TGFβ or its ligand.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.

Figure 1 is a plot showing concentration of VEGF secreted by monocytic cells, endothelial cells and RA fibroblasts after stimulation in absence or presence of TNFα or IL-1α, as determined by ELISA. Values are means of 3 determinations ±SD, and are representative of 3 similar experiments.

Figure 2A is a plot showing serum VEGF concentrations in age- and sex-matched non-arthritic individuals and in patients with RA, as measured by ELISA.

Figure 2B is a plot showing the degree of correlation between serum VEGF concentrations and C-reactive protein levels.

Figure 3 is graph showing serum VEGF levels in RA patients after infusion of either placebo, 1 mg/kg anti-TNFα antibody cA2 or 10 mg/Rg anti-TNFα antibody cA2, as determined by ELISA.

Figure 4A is a graph showing serum VEGF levels in RA patients after treatment with either anti-TNFα antibody cA2 at 3 mg/kg (infusions at weeks 0, 2, 6, 10 and 14), methotrexate (7.5 mg/week) or a combination of cA2 and methotrexate.

Figure 4B is a graph showing serum VEGF levels in RA patients after treatment with methotrexate (7.5 mg/week), either alone or in combination with anti-TNFα antibody cA2 at either 1 mg/kg, 3 mg/kg or 10 mg/kg (infusions at weeks 0, 2, 6, 10 and 14).

### DETAILED DESCRIPTION OF THE INVENTION

The work described herein shows that production of VEGF is inducible by pro-inflammatory cytokines such as TNFα and IL-1, and that blockade of TNFα activity *in vivo* decreases circulating concentrations of VEGF. In a disease associated with angiogenesis, it is likely that excess VEGF present in the circulation is produced in the diseased tissue. Since VEGF is a potent and specific inducer of angiogenesis, reduction of circulating VEGF reflects a reduced predisposition to angiogenesis. Therefore, long term blockade of TNFα can reduce neovascularisation and hence the cellular mass in the diseased tissue.

A prominent feature of rheumatoid arthritis lesions is an infiltrate of inflammatory cells from the blood, together with invading pannus which is associated with prominent new blood formation, thus perpetuating the ingress of nutrients and cells, and the inflammatory reactions which culminate in bone and cartilage destruction. VEGF is a potent inducer of angiogenesis and has been implicated in the formation of blood vessels and activation of microvascular endothelium in RA.

The data described herein show that VEGF serum concentrations are significantly elevated in patients with active RA, relative to VEGF serum concentrations in normal individuals, and correlate with C-reactive protein (CRP), a marker of inflammation and disease activity in RA. The production of CRP is regulated by pro-inflammatory cytokines, and its overall production correlates well with the rate of disease progression. Moreover, treatment of RA patients with anti-TNFα monoclonal antibody, which leads to amelioration of disease symptoms, results in a significant and persistent reduction in serum VEGF concentrations. Treatment of RA patients with a combination of anti-TNFα monoclonal antibody and methotrexate results in a more prolonged reduction in serum VEGF levels relative to patients treated with anti-TNFα antibody alone or with methotrexate alone. It is likely that serum VEGF reflects synthesis of VEGF, thus suggesting that VEGF production *in vivo* is cytokine-dependent.

VEGF results in the induction of increased vascular permeability and the leakage of vascular fluid into surrounding tissues. In the rheumatoid joint, the presence of extravascular fluid is well documented and is clinically apparent as joint effusion. Moreover, it has also been confirmed that treatment of RA patients with anti-TNFα antibody results in a reduction in joint fluid content as determined by MRI imaging (Kalden-Nemeth *et al., Rheumatol. Int., 16*:219 (1997)). This suggests that VEGF also plays a role in joint swelling in RA, which is rapidly reduced after treatment with anti-TNFα.

The data also show that monocytic cells, endothelial cells and synovial membrane fibroblasts, which may be hypothesised to contribute to elevated serum VEGF concentrations in RA, secrete VEGF constitutively, and that spontaneous release of VEGF by RA synovial membrane cells is markedly reduced in the presence of inhibitors of cytokine activity, namely anti-TNFα antibody and IL-1 receptor antagonist (IL-1ra). The reduction in VEGF secretion from synovial membrane cells by IL-1ra and anti-TNFα antibody provides evidence that serum VEGF production *in vivo* is regulated by pro-inflammatory cytokines.

Isolation of RA synovial membrane cells by collagenase/DNase digestion yields a heterogeneous population of cells, consisting predominantly of mononuclear cells and to a lesser extent fibroblasts (Brennan *et al., Lancet, 2*:244-247 (1989); and Buchan *et al., Clin. Exp. Immunol., 73*:449-455 (1988)). Long-term (up to 9 days) culture of these cells leads to decreased numbers of CD14+, CD45+ and CD3+ cells, accompanied by the appearance of adherent fibroblast-like cells. The earliest detectable release of VEGF into cell culture supernatants was observed at 24 hours. However, VEGF levels in culture continued to increase even 9 days after isolation, which contrasts with the rapid increase (within 2 hours of plating) in TNFα levels in culture supernatants of RA synovial membrane cells, which subsequently returned to undetectable levels by days 5-6 of culture (Buchan *et al., Clin. Exp. Immunol., 73*:449-455 (1988)). Although macrophage-like cells are the predominant source of TNFα in RA synovial membrane explant cultures, VEGF is released both by monocytic cells and fibroblasts. This is in agreement with the data described herein using THP-1 cells and synovial fibroblasts.

The differential *in vitro* sensitivity of RA synovial membrane fibroblasts, endothelial cells and monocytic cells to TNFα and IL-1, in terms of VEGF secretion, suggests that the reduction in serum VEGF is a result of decreased VEGF production by monocyte/ macrophages and microvascular endothelial cells following *in vivo* blockade of TNFα activity by anti-TNFα antibody and of IL-1 activity subsequent to TNFα blockade. Synovial explant cultures consist of both macrophage-like cells (which release VEGF in response to TNFα) and fibroblasts (secretion from which is induced only by IL-1). These cells contribute to differing degrees to VEGF release into the cell supernatants. Thus, optimal inhibition of VEGF production in RA patients may require the blockade of both TNFα and IL-1 activities.

There may be an additional indirect effect on release of VEGF by fibroblasts, since although synovial membrane fibroblasts failed to secrete significant amounts of VEGF in response to TNFα, IL-1 was found to be a potent stimulus for these cells, and anti-TNFα antibody treatment also deactivates the cascade of cytokines downstream of TNFα (Brennan *et al., Lancet, 2*:244-247 (1989)). The incomplete reduction by anti-TNFα antibody of serum VEGF concentrations is most probably due to the presence *in vivo* of a cytokine-independent component of VEGF production, such as that due to hypoxia (Blake *et al., Lancet, 8633*:289-293 (1989); and Tuder *et al., J. Clin. Invest., 95*:1798-1807 (1995)). Moreover, monocytic cells, endothelial cells and fibroblasts release significant amounts of VEGF in the absence of extrinsic stimulus.

The results clearly indicate that pro-inflammatory cytokines, including TNFα and IL-1, are involved in the regulation of VEGF production, *in vitro* and *in vivo.* In particular, as judged by the decrease in serum concentrations after anti-TNFα antibody treatment, TNFα modulates production of VEGF *in vivo,* suggesting that part of the benefit of anti-TNFα antibody therapy may be due to reductions in angiogenesis, and that long term TNFα blockade can reduce neovascularisation and hence, the cellular mass of the pannus and its destructive potential.

Thus, VEGF and other components of the angiogenic pathway are appropriate targets in RA for achieving synergism with anti-TNFα, therapy. Thus, the invention includes methods for treating and/or preventing a TNF-mediated disease in an individual, comprising co-administering a TNFα antagonist and a VEGF antagonist to the individual in therapeutically effective or synergistic amounts. The present invention further relates to a method for treating and/or preventing recurrence of a TNF-mediated disease in an individual comprising co-administering a TNFα antagonist and a VEGF antagonist to the individual in therapeutically effective amounts. The TNFα antagonist and VEGF antagonist can be administered simultaneously or sequentially. The TNFα antagonist and VEGF antagonist can each be administered in single or multiple doses. Multiple VEGF antagonists and multiple TNF antagonists can be co-administered. Other therapeutic regimens and agents can be used in combination with the therapeutic co-administration of TNFα antagonists and VEGF antagonists. For example, in a particular embodiment, methotrexate is co-administered with the TNFα antagonist and the VEGF antagonist in therapeutically effective or synergistic amounts.

As used herein, a "TNF-mediated disease" refers to a TNF related pathology or disease. TNF related pathologies or diseases include, but are not limited to, the following:
(A) inflammatory diseases, including, but not limited to, acute and chronic immune and autoimmune pathologies, such as, but not limited to, rheumatoid arthritis (RA), juvenile chronic arthritis (JCA), spondyloarthropathy, thyroiditis, graft versus host disease (GVHD), scleroderma, diabetes mellitus, Graves' disease, allergy; acute or chronic immune disease associated with an allogenic transplantation, such as, but not limited to, renal transplantation, cardiac transplantation, bone marrow transplantation, liver transplantation, pancreatic transplantation, small intestine transplantation, lung transplantation and skin transplantation; chronic inflammatory pathologies such as, but not limited to, sarcoidosis, chronic inflammatory bowel disease, ulcerative colitis, and Crohn's pathology or disease; vascular inflammatory pathologies, such as, but not limited to, disseminated intravascular coagulation, atherosclerosis, Kawasaki's pathology and vasculitis syndromes, such as, but not limited to, polyarteritis nodosa, Wegener's granulomatosis, Henoch-Schonlein purpura, giant cell arthritis and microscopic vasculitis of the kidneys; chronic active hepatitis; Sjögren's syndrome; spondyloarthropathies, such as ankylosing spondylitis, psoriatic arthritis and spondylitis, enteropathic arthritis and spondylitis, reactive arthritis and arthritis associated with inflammatory bowel disease; and uveitis;
(B) infections, including, but not limited to, sepsis syndrome, cachexia, circulatory collapse and shock resulting from acute or chronic bacterial infection, acute and chronic parasitic and/or infectious diseases, bacterial, viral or fungal, such as a human immunodeficiency virus (HIV), acquired immunodeficiency syndrome (AIDS) (including symptoms of cachexia, autoimmune disorders, AIDS dementia complex and infections) ;
(C) neurodegenerative diseases, including, but not limited to, demyelinating diseases, such as multiple sclerosis and acute transverse myelitis; myasthenia gravis; extrapyramidal and cerebellar disorders, such as lesions of the corticospinal system; disorders of the basal ganglia or cerebellar disorders; hyperkinetic movement disorders, such as Huntington's chorea and senile chorea; drug-induced movement disorders, such as those induced by drugs which block central nervous system (CNS) dopamine receptors; hypokinetic movement disorders, such as Parkinson's disease; progressive supranuclear palsy; cerebellar and spinocerebellar disorders, such as astructural lesions of the cerebellum; spinocerebellar degenerations (spinal ataxia, Friedreich's ataxia, cerebellar cortical degenerations, multiple systems degenerations (Mencel, Dejerine-Thomas, Shi-Drager, and MachadoJoseph)); and systemic disorders (Refsum's disease, abetalipoproteinemia, ataxia, telangiectasia, and mitochondrial multisystem disorder); disorders of the motor unit, such as neurogenic muscular atrophies (anterior horn cell degeneration, such as amyotrophic lateral sclerosis, infantile spinal muscular atrophy and juvenile spinal muscular atrophy); Alzheimer's disease; Down's syndrome in middle age; diffuse Lewy body disease; senile dementia of Lewy body type; Wernicke-Korsakoff syndrome; chronic alcoholism; primary biliary cirrhosis; cryptogenic fibrosing alveolitis and other fibrotic lung diseases; hemolytic anemia; Creutzfeldt-Jakob disease; subacute sclerosing panencephalitis, Hallerrorden-Spatz disease; and dementia pugilistica, or any subset thereof;
(D) malignant pathologies involving TNFα-secreting tumors or other malignancies involving TNFα, such as, but not limited to, leukemias (acute, chronic myelocytic, chronic lymphocytic and/or myelodyspastic syndrome); lymphomas (Hodgkin's and non-Hodgkin's lymphomas, such as malignant lymphomas (Burkitt's lymphoma or Mycosis fungoides));
(E) cachectic syndromes and other pathologies and diseases involving excess TNFα, such as, but not limited to, cachexia of cancer, parasitic disease and heart failure;
(F) alcohol-induced hepatitis and other forms of chronic hepatitis; and
(G) diseases in which angiogenesis or VEGF/VPF production plays a part, such as, but not limited to, ocular neovascularization, psoriasis, duodenal ulcers, angiogenesis of the female reproductive tract, and chronic arthritis, including osteoarthritis vilonodular synovitis, and chronic arthritis associated with hemorraghic diseases, such as hemophilic arthritis.

See, e.g., Berkow *et al.,* Eds., *The Merck Manual,* 16th edition, Chapter 11, pp. 1380-1529, Merck and Co., Rahway, New Jersey, 1992, incorporated herein by reference.

The terms "recurrence", "flare-up" or "relapse" are defined to encompass the reappearance of one or more symptoms of the disease state. For example, in the case of rheumatoid arthritis, a recurrence can include the experience of one or more of swollen joints, morning stiffness or joint tenderness.

In one embodiment, the invention relates to a method of treating and/or preventing rheumatoid arthritis in an individual comprising co-administering a TNFα antagonist and a VEGF antagonist to the individual in therapeutically effective or synergistic amounts.

In a second embodiment, the invention relates to a method for treating and/or preventing Crohn's disease in an individual comprising co-administering a TNFα antagonist and a VEGF antagonist to the individual in therapeutically effective or synergistic amounts.

In a third embodiment, the invention relates to a method for treating and/or preventing an acute or chronic immune disease associated with an allogenic transplantation in an individual comprising co-administering a TNFα antagonist and a VEGF antagonist to the individual in therapeutically effective or synergistic amounts. As used herein, a "transplantation" includes organ, tissue or cell transplantation, such as renal transplantation, cardiac transplantation, bone marrow transplantation, liver transplantation, pancreatic transplantation, small intestine transplantation, skin transplantation and lung transplantation.

In a particular embodiment, the methods of the invention further comprise administering methotrexate to the individual in a therapeutically effective or synergistic amount.

A benefit of combination therapy with a TNFα antagonist and a VEGF antagonist is significantly improved response in comparison with that obtained with treatment with each antagonist alone. Combination therapy with a TNFα antagonist, methotrexate and a VEGF antagonist also provides a significantly improved response in comparison with that obtained with treatment with each agent alone. For example, a VEGF antagonist can be administered in combination with a TNFα antagonist or with a TNFα antagonist and methotrexate to achieve a synergistic effect. In addition, lower dosages can be used to provide the same therapeutic response, thus increasing the therapeutic window between a therapeutic and a toxic effect. Lower doses may also result in lower financial costs to the patient, and potentially fewer side effects. Further, methotrexate reduces immunogenicity of anti-TNFα antibodies, thus permitting administration of anti-TNFα antibodies with enhanced safety.

The invention also relates to compositions comprising a TNFα antagonist and a VEGF antagonist. In a particular embodiment, the compositions further comprise methotrexate. The compositions of the present invention are useful for treating a subject having a pathology or condition associated with abnormal levels of a substance reactive with a TNFα antagonist, in particular TNFα in excess of levels present in a normal healthy subject, where such excess or diminished levels occur in a systemic, localized or particular tissue type or location in the body. Such tissue types can include, but are not limited to, blood, lymph, central nervous system (CNS), liver, kidney, spleen, heart muscle or blood vessels, brain or spinal cord white matter or grey matter, cartilage, ligaments, tendons, lung, pancreas, ovary, testes, prostate. Increased TNFα concentrations relative to normal levels can also be localized to specific regions or cells in the body, such as joints, nerve blood vessel junctions, bones, specific tendons or ligaments, or sites of infection, such as bacterial or viral infections. The compositions of the present invention can also be used in the manufacture of a medicament for treating the above diseases.

### Tumor Necrosis Factor Alpha Antagonists

As used herein, a "tumor necrosis factor alpha antagonist" decreases, blocks, inhibits, abrogates or interferes with TNFα activity *in vivo.* For example, a suitable TNFα antagonist can bind TNFα and includes anti-TNFα antibodies, antigen-binding fragments thereof, and receptor molecules and derivatives which bind specifically to TNFα. A suitable TNFα antagonist can also prevent or inhibit TNFα synthesis and/or TNFα release and includes compounds such as thalidomide, tenidap, and phosphodiesterase inhibitors, such as, but not limited to, pentoxifylline and rolipram. A suitable TNFα antagonist that can prevent or inhibit TNFα synthesis and/or TNFα release also includes A2b adenosine receptor enhancers and A2b adenosine receptor agonists (e.g., 5'-(N-cyclopropyl)-carboxamidoadenosine, 5'-N-ethylcarboxamidoadenosine, cyclohexyladenosine and R-N⁶-phenyl-2-propyladenosine). See, for example, Jacobson, GB 2 289 218 A, the teachings of which are entirely incorporated herein by reference. A suitable TNFα antagonist can also prevent or inhibit TNFα receptor signalling and includes mitogen activated protein (MAP) kinase inhibitors (e.g., SB 203580; Lee and Young, *J. Leukocyte Biol.*, *59*:152-157 (1996), the teachings of which are entirely incorporated herein by reference). Other suitable TNFα antagonists include agents which decrease, block, inhibit, abrogate or interfere with membrane TNFα cleavage, such as, but not limited to, metalloproteinase inhibitors; agents which decrease, block, inhibit, abrogate or interfere with TNFα activity, such as, but not limited to, angiotensin converting enzyme (ACE) inhibitors, such as captopril, enalapril and lisinopril; and agents which decrease, block, inhibit, abrogate or interfere with TNFα production and/or synthesis, such as, but not limited to, MAP kinase inhibitors. TNFα antagonists are also described in U.S. Application No. 08/690,775 (filed August 1, 1996), U.S. Application No. 08/607,419 (filed February 28, 1996), International Publication No. WO 95/09652 (published April 13, 1995), U.S. Application No. 08/403,785 (filed October 6, 1993), International Publication No. WO 94/08619 (published April 28, 1994), U.S. Application No. 07/958,248 (filed October 8, 1992). These references are all entirely incorporated herein by reference.

### Anti-TNFα Antibodies

As used herein, an anti-tumor necrosis factor alpha antibody decreases, blocks, inhibits, abrogates or interferes with TNFα activity *in vivo.* In a preferred embodiment, the antibody specifically binds the antigen. The antibody can be polyclonal or monoclonal, and the term antibody is intended to encompass both polyclonal and monoclonal antibodies. The terms polyclonal and monoclonal refer to the degree of homogeneity of an antibody preparation, and are not intended to be limited to particular methods of production.

Suitable antibodies are available, or can be raised against an appropriate immunogen, such as isolated and/or recombinant antigen or portion thereof (including synthetic molecules, such as synthetic peptides) or against a host cell which expresses recombinant antigen. In addition, cells expressing recombinant antigen, such as transfected cells, can be used as immunogens or in a screen for antibody which binds receptor (see e.g., Chuntharapai *et al., J. Immunol., 152*: 1783-1789 (1994); and Chuntharapai *et al.,* U.S. Patent No. 5,440,021).

Preparation of immunizing antigen, and polyclonal and monoclonal antibody production can be performed using any suitable technique. A variety of methods have been described (see e.g., Kohler *et al., Nature, 256:* 495-497 (1975) and *Eur*. *J. Immunol., 6:* 511-519 (1976) ; Milstein *et al., Nature, 266: 550-552 (1977);* Koprowski *et al.,* U.S. Patent No. 4,172,124; Harlow, E. and D. Lane, 1988, *Antibodies: A Laboratory Manual,* (Cold Spring Harbor Laboratory: Cold Spring Harbor, NY); and *Current Protocols In Molecular Biology,* Vol. 2 (Supplement 27, Summer '94), Ausubel *et al.,* Eds., (John Wiley & Sons: New York, NY), Chapter 11, (1991)). Generally, a hybridoma can be produced by fusing a suitable immortal cell line (e.g., a myeloma cell line such as SP2/0) with antibody producing cells. The antibody producing cell, preferably those of the spleen or lymph nodes, can be obtained from animals immunized with the antigen of interest. The fused cells (hybridomas) can be isolated using selective culture conditions, and cloned by limiting dilution. Cells which produce antibodies with the desired specificity can be selected by a suitable assay (e.g., ELISA).

Other suitable methods of producing or isolating antibodies of the requisite specificity, including human antibodies, can be used, including, for example, methods by which a recombinant antibody or portion thereof are selected from a library, such as, for example, by phage display technology (see, e.g., Winters *et al., Annu. Rev. Immunol., 12*:433-455 (1994); Hoogenboom *et al.,* WO 93/06213; Hoogenboom et *al.,* U.S. Patent No. 5,565,332; WO 94/13804, published June 23, 1994; Krebber *et al.,* U.S. Patent No. 5,514,548; and Dower *et al.,* U.S. Patent No. 5,427,908), or which rely upon immunization of transgenic animals (e.g., mice) capable of producing a full repertoire of human antibodies (see e.g., Jakobovits *et al., Proc. Natl. Acad. Sci. USA, 90*: 2551-2555 (1993); Jakobovits *et al., Nature, 362*:255-258 (1993); Kucherlapati *et al.,* European Patent No. EP 0 463 151 B1; Lonberg *et al.,* U.S. Patent No. 5,569,825; Lonberg *et al.,* U.S. Patent No. 5,545,806; and Surani *et al.,* U.S. Patent No. 5,545,807).

Single chain antibodies, and chimeric, humanized or primatized (CDR-grafted antibodies, with or without framework changes), or veneered antibodies, as well as chimeric, CDR-grafted or veneered single chain antibodies, comprising portions derived from different species, and the like are also encompassed by the present invention and the term "antibody". The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., Cabilly *et al.,* U.S. Patent No. 4,816,567; Cabilly *et al.,* European Patent No. 0,125,023 B1; Boss *et al.,* U.S. Patent No. 4,816,397; Boss *et al.,* European Patent No. 0,120,694 B1; Neuberger, M.S. *et al.,* WO 86/01533; Neuberger, M.S. *et al.,* European Patent No. 0,194,276 B1; Winter, U.S. Patent No. 5,225,539; Winter, European Patent No. 0,239,400 B1; Queen *et al.,* U.S. Patent No. 5,585,089; Queen *et al.,* European Patent No. 0,451,216 B1; Adair *et al.,* WO 91/09967, published 11 July 1991; Adair *et al.,* European Patent No. 0,460,167 B1; and Padlan, E.A. *et al.,* European Patent No. 0,519,596 A1. See also, Newman, R. *et al., BioTechnology, 10:* 1455-1460 (1992), regarding primatized antibody, and Huston *et al.,* U.S. Patent No. 5,091,513; Huston *et al.,* U.S. Patent No. 5,132,405; Ladner *et al.,* U.S. Patent No. 4,946,778 and Bird, R.E. *et al., Science, 242:* 423-426 (1988)) regarding single chain antibodies.

In addition, antigen binding fragments of antibodies, including fragments of chimeric, humanized, primatized, veneered or single chain antibodies and the like, can also be produced. For example, antigen binding fragments include, but are not limited to, fragments such as Fv, Fab, Fab' and F(ab')₂ fragments. Antigen binding fragments can be produced by enzymatic cleavage or by recombinant techniques, for example. For instance, papain or pepsin cleavage can generate Fab or F(ab')₂ fragments, respectively. Antibodies can also be produced in a variety of truncated forms using antibody genes in which one or more stop codons has been introduced upstream of the natural stop site. For example, a chimeric gene encoding a F(ab')₂ heavy chain portion can be designed to include DNA sequences encoding the CH₁ domain and hinge region of the heavy chain.

Anti-TNFα antibodies useful in the present invention are characterized by high affinity binding to TNFα and low toxicity (including human anti-murine antibody (HAMA) and/or human anti-chimeric antibody (HACA) response). In particular, an antibody where the individual components, such as the variable region, constant region and framework, individually and/or collectively possess low immunogenicity is useful in the present invention. The antibodies which can be used in the invention are characterized by their ability to treat patients for extended periods with good to excellent alleviation of symptoms and low toxicity. Low immunogenicity and/or high affinity, as well as other undefined properties, may contribute to the therapeutic results achieved. "Low immunogenicity" is defined herein as raising significant HACA or HAMA responses in less than about 75%, or preferably less than about 50% of the patients treated and/or raising low titres in the patient treated (less than about 300, preferably less than about 100 measured.with a double antigen enzyme immunoassay) (Elliott *et al., Lancet 344*:1125-1127 (1994), incorporated herein by reference).

In a particular embodiment, the anti-TNFα antibody is chimeric monoclonal antibody cA2 (or an antigen binding fragment thereof) or murine monoclonal antibody A2 (or an antigen binding fragment thereof), or has an epitopic specificity similar to that of chimeric antibody cA2, murine monoclonal antibody A2, or antigen binding fragments thereof, including antibodies or antigen binding fragments reactive with the same or a functionally equivalent epitope on human TNFα as that bound by chimeric antibody cA2 or murine monoclonal antibody A2, or antigen binding fragments thereof. Antibodies with an epitopic specificity similar to that of chimeric antibody cA2 or murine monoclonal antibody A2 include antibodies which can compete with chimeric antibody cA2 or murine monoclonal antibody A2 (or antigen binding fragments thereof) for binding to human TNFα. Such antibodies or fragments can be obtained as described above. Chimeric antibody cA2, murine monoclonal antibody A2 and methods of obtaining these antibodies are also described in U.S. Application No. 08/192,093 (filed February 4, 1994), U.S. Application No. 08/192,102 (filed February 4, 1994), U.S. Application No. 08/192,861 (filed February 4, 1994), U.S. Application No. 08/324,799 (filed October 18, 1994), Le, J. *et al.,* International Publication No. WO 92/16553 (published October 1, 1992), Knight, D.M. *et al., Mol. Immunol., 30*:1443-1453 (1993), and Siegel, S.A. *et al., Cytokine,* 7(1):15-25 (1995), which references are each entirely incorporated herein by reference.

Chimeric antibody cA2 consists of the antigen binding variable region of the high-affinity neutralizing mouse anti-human TNFα IgGl antibody, designated A2, and the constant regions of a human IgG1, kappa immunoglobulin. The human IgG1 Fc region improves allogeneic antibody effector function, increases the circulating serum half-life and decreases the immunogenicity of the antibody. The avidity and epitope specificity of the chimeric antibody cA2 is derived from the variable region of the murine antibody A2. In a particular embodiment, a preferred source for nucleic acids encoding the variable region of the murine antibody A2 is the A2 hybridoma cell line.

Chimeric A2 (cA2) neutralizes the cytotoxic effect of both natural and recombinant human TNFα in a dose dependent manner. From binding assays of chimeric antibody cA2 and recombinant human TNFα, the affinity constant of chimeric antibody cA2 was calculated to be 1.04x10¹⁰M⁻¹. Preferred methods for determining monoclonal antibody specificity and affinity by competitive inhibition can be found in Harlow, *et al., Antibodies: A Laboratory Manual,* Cold Spring Harbor Laboratory Press, Cold Spring Harbor, New York, 1988; Colligan *et al.,* eds., *Current Protocols in Immunology,* Greene Publishing Assoc. and Wiley Interscience, New York, (1992, 1993); Kozbor *et al., Immunol. Today, 4*:72-79 (1983); Ausubel *et al.,* eds. *Current Protocols in Molecular Biology,* Wiley Interscience, New York (1987, 1992, 1993); and Muller, *Meth. Enzymol., 92*:589-601 (1983), which references are entirely incorporated herein by reference.

In a particular embodiment, murine monoclonal antibody A2 is produced by a cell line designated c134A. Chimeric antibody cA2 is produced by a cell line designated c168A.

Additional examples of anti-TNFα antibodies (or antigen-binding fragments thereof) are described in the art (see, e.g., U.S. Patent No. 5,231,024; Möller, A. *et al., Cytokine, 2*(3):162-169 (1990); U.S. Application No. 07/943,852 (filed September 11, 1992); Rathjen *et al.,* International Publication No. WO 91/02078 (published February 21, 1991); Rubin *et al.,* EPO Patent Publication No. 0 218 868 (published April 22, 1987); Yone *et al.,* EPO Patent Publication No. 0 288 088 (October 26, 1988); Liang, *et al., Biochem. Biophys. Res. Comm., 137*:847-854 (1986); Meager, *et al., Hybridoma, 6*:305-311 (1987); Fendly *et al., Hybridoma,* *6*:359-369 (1987); Bringman, *et al., Hybridoma, 6*:489-507 (1987); and Hirai, *et al., J*. *Immunol. Meth., 96*:57-62 (1987), which references are entirely incorporated herein by reference).

As used herein, the term "antigen binding region" refers to that portion of an antibody molecule which contains the amino acid residues that interact with an antigen and confer on the antibody its specificity and affinity for the antigen. The antigen binding region includes the "framework" amino acid residues necessary to maintain the proper conformation of the antigen-binding residues.

The term antigen refers to a molecule or a portion of a molecule capable of being bound by an antibody which is additionally capable of inducing an animal to produce antibody capable of selectively binding to an epitope of that antigen. An antigen can have one or more than one epitope.

The term epitope is meant to refer to that portion of the antigen capable of being recognized by and bound by an antibody at one or more of the antibody's antigen binding region. Epitopes usually consist of chemically active surface groupings of molecules such as amino acids or sugar side chains and have specific three dimensional structural characteristics as well as specific charge characteristics. By "inhibiting and/or neutralizing epitope" is intended an epitope, which, when bound by an antibody, results in loss of biological activity of the molecule containing the epitope, *in vivo* or *in vitro,* more preferably *in vivo,* including binding of TNFα to a TNFα receptor.

### TNFα Receptor Molecules

TNFα receptor molecules useful in the methods and compositions of the present invention are those that bind TNFα with high affinity (see, e.g., Feldmann *et al.,* International Publication No. WO 92/07076 (published April 30, 1992); Schall *et al., Cell, 61*:361-370 (1990); and Loetscher *et al., Cell, 61*:351-359 (1990), which references are entirely incorporated herein by reference) and possess low immunogenicity. In particular, the 55 kDa (p55 TNF-R) and the 75 kDa (p75 TNF-R) TNFα cell surface receptors are useful in the present invention. Truncated forms of these receptors, comprising the extracellular domains (ECD) of the receptors or functional portions thereof (see, e.g., Corcoran *et al., Eur. J*. *Biochem., 223*:831-840 (1994)), are also useful in the present invention. Truncated forms of the TNFα receptors, comprising the ECD, have been detected in urine and serum as 30 kDa and 40 kDa TNFα inhibitory binding proteins (Engelmann, H. *et al., J. Biol. Chem., 265*:1531-1536 (1990)). TNFα receptor multimeric molecules and TNFα immunoreceptor fusion molecules, and derivatives and fragments or portions thereof, are additional examples of TNFα receptor molecules which are useful in the methods and compositions of the present invention. TNFα receptor molecules which can be used in the invention are characterized by their ability to treat patients for extended periods with good to excellent alleviation of symptoms and low toxicity. Low immunogenicity and/or high affinity, as well as other undefined properties, may contribute to the therapeutic results achieved.

TNFα receptor multimeric molecules useful in the present invention comprise all or a functional portion of the ECD of two or more TNFα receptors linked via one or more polypeptide linkers or other nonpeptide linkers, such as polyethylene glycol (PEG). The multimeric molecules can further comprise a signal peptide of a secreted protein to direct expression of the multimeric molecule. These multimeric molecules and methods for their production have been described in U.S. Application No. 08/437,533 (filed May 9, 1995), the content of which is entirely incorporated herein by reference.

TNFα immunoreceptor fusion molecules useful in the methods and compositions of the present invention comprise at least one portion of one or more immunoglobulin molecules and all or a functional portion of one or more TNFα receptors. These immunoreceptor fusion molecules can be assembled as monomers, or hetero- or homo-multimers. The immunoreceptor fusion molecules can also be monovalent or multivalent. An example of such a TNFα immunoreceptor fusion molecule is TNFα receptor/IgG fusion protein.

TNFα immunoreceptor fusion molecules and methods for their production have been described in the art (Lesslauer *et al., Eur. J. Immunol., 21*:2883-2886 (1991); Ashkenazi *et al., Proc. Natl. Acad. Sci. USA, 88*:10535-10539 (1991); Peppel *et al., J. Exp. Med., 174*:1483-1489 (1991); Kolls *et al., Proc. Natl. Acad. Sci. USA, 91*:215-219 (1994); Butler *et al., Cytokine, 6*(6):616-623 (1994); Baker *et al.*, *Eur. J. Immunol., 24*:2040-2048 (1994); Beutler *et al.,* U.S. Patent No. 5,447,851; and U.S. Application No. 08/442,133 (filed May 16, 1995), which references are entirely incorporated herein by reference). Methods for producing immunoreceptor fusion molecules can also be found in Capon *et al.,* U.S. Patent No. 5,116,964; Capon *et al.,* U.S. Patent No. 5,225,538; and Capon *et al., Nature, 337*:525-531 (1989), which references are entirely incorporated herein by reference.

A functional equivalent, derivative, fragment or region of TNFα receptor molecule refers to the portion of the TNFα receptor molecule, or the portion of the TNFα receptor molecule sequence which encodes TNFα receptor molecule, that is of sufficient size and sequences to functionally resemble TNFα receptor molecules that can be used in the present invention (e.g., bind TNFα with high affinity and possess low immunogenicity). A functional equivalent of TNFα receptor molecule also includes modified TNFα receptor molecules that functionally resemble TNFα receptor molecules that can be used in the present invention (e.g., bind TNFα with high affinity and possess low immunogenicity). For example, a functional equivalent of TNFα receptor molecule can contain a "SILENT" codon or one or more amino acid substitutions, deletions or additions (e.g., substitution of one acidic amino acid for another acidic amino acid; or substitution of one codon encoding the same or different hydrophobic amino acid for another codon encoding a hydrophobic amino acid). See Ausubel *et al., Current Protocols in Molecular Biology,* Greene Publishing Assoc. and Wiley-Interscience, New York (1989).

### VEGF Antagonists

As used herein, a "vascular endothelial growth factor antagonist" decreases, blocks, inhibits, abrogates or interferes with VEGF activity synthesis or receptor signalling *in vivo.* VEGF antagonists include anti-VEGF antibodies and antigen-binding fragments thereof; receptor molecules and derivatives which bind specifically to VEGF; and VEGF receptor antagonists. VEGF antagonists include agents which decrease, inhibit, block, abrogate or interfere with VEGF function, such as, but not limited to, suramin and protein tyrosine kinase (PTK) inhibitors (e.g., lavendustin A). See, e.g., Waltenberger *et al., J. Mol. Cell. Cardiol., 28*:1523-1529 (1996); and Hu *et al., Brit. J. Pharmacol., 114*:262-268 (1995). VEGF antagonists also include agents which decrease, inhibit, block, abrogate or interfere with binding of VEGF to VEGF receptors or extracellular domains thereof, such as, but not limited to, platelet factor-4 (PF-4). See, e.g., Gengrinovitch *et al., J. Biol. Chem., 270*:15059-15065 (1995). VEGF antagonists include agents which decrease, inhibit, block, abrogate or interfere with VEGF receptor signalling; and agents which decrease, inhibit, block, abrogate or interfere with VEGF activation, such as, but not limited to, mithramycin. See, e.g., Ryuto *et al., J. Biol. Chem., 271*(45):28220-28228 (1996). VEGF antagonists also include agents which decrease, inhibit, block, abrogate or interfere with VEGF production, such as compounds (e.g. drugs and other agents, including antibodies) which inhibit, block, abrogate or interfere with TGFβ or its ligands. See, e.g., Frank *et al., J. Biol. Chem., 270*:12607-12613 (1995); and Pertovaara et *al., J. Biol. Chem., 269*:6271-6274 (1994). VEGF antagonists further include agents which are antagonists of signals that drive VEGF production and/or synthesis.

### Anti-VEGF Antibodies

As used herein, an anti-VEGF antibody (or an antigen binding fragment thereof) decreases, blocks, inhibits, abrogates or interferes with VEGF activity *in vivo*. Antibodies and antigen binding fragments are as described above.. For example, the antibody can be polyclonal or monoclonal. As above, the antibody can be a single chain chimeric, humanized, primatized or veneered antibody. Such antibodies or fragments can be obtained as described above. Advantageously, anti-VEGF antibodies (and antigen binding fragments thereof) are characterized by high affinity binding to VEGF (such as high affinity binding to VEGF₁₂₁, VEGF₁₆₅, VEGF₁₈₉ or VEGF₂₀₆) and low toxicity (including HAMA and/or HACA response). An antibody where the individual components, such as the variable region, constant region and framework, individually and/or collectively possess low immunogenicity is particularly useful. In a particular embodiment, anti-VEGF antibodies (and antigen binding fragments thereof) are characterized by their ability to treat patients for extended periods with good to excellent alleviation of symptoms and low toxicity. Low immunogenicity and/or high affinity, as well as other undefined properties, may contribute to the therapeutic results achieved.

Examples of anti-VEGF antibodies (or antigen binding fragments thereof) are described in the art (see, e.g., Asano *et al., Hybridoma, 14*(5):475-480 (1955); and Kim *et al., Growth Factors, 7*:53-64 (1992), which references are entirely incorporated herein by reference).

### VEGF Receptor Molecules

VEGF receptor molecules useful in the present invention bind specifically to VEGF and possess low immunogenicity. Preferably, the VEGF receptor molecule is characterized by high affinity binding to VEGF. VEGF receptor molecules include VEGF receptors, such as tyrosine kinase receptors, KDR, Flk (e.g., Flk-1) and Flt (e.g., Flt-1 and Flt-4) (see, e.g., *Lee et al., Proc. Natl. Acad. Sci. USA, 93*:1988-1992 (1996); deVries *et al., Science, 255*:989-991 (1992); Quinn *et al., Proc. Natl. Acad. Sci. USA, 90*:7533-7537 (1993); Shibuya *et al., Oncogene, 5*:519-524 (1990); and Terman *et al., Biochem. Biophys. Res. Commun., 187*:1579-1586 (1992), which references are entirely incorporated herein by reference). VEGF receptor molecules also include VEGF receptor multimeric molecules and VEGF immunoreceptor fusion molecules, and derivatives and fragments or portions thereof.

VEGF receptor multimeric molecules can comprise all or a functional portion of two or more VEGF receptors linked via one or more linkers. VEGF receptor multimeric molecules can further comprise a signal peptide of a secreted protein to direct expression of the multimeric molecule.

VEGF immunoreceptor fusion molecules can comprise at least one portion of one or more immunoglobulin molecules and all or a functional portion of one or more VEGF receptor(s). VEGF immunoreceptor fusion molecules can be assembled as monomers, or hetero- or homo-multimers. VEGF immunoreceptor fusion molecules can also be monovalent or multivalent. Examples of VEGF immunoreceptor fusion molecules are described by Aiello *et al., Proc. Natl. Acad. Sci. USA, 92*(23):10457-10461 (1995), the teaching of which is entirely incorporated herein by reference. See also, Aiello *et al., N. Engl. J. Med., 331*:1480-1487 (1994); and Park *et al., J. Biol. Chem., 269*:25646-25654 (1994), the teachings of which are entirely incorporated herein by reference.

A functional equivalent, derivative, fragment or region of VEGF receptor molecule refers to the portion of the VEGF receptor molecule, or the portion of the VEGF receptor molecule sequence which encodes VEGF receptor molecule, that is of sufficient size and sequences to functionally resemble VEGF receptor molecules that can be used in the present invention (e.g., bind specifically to VEGF and possess low immunogenicity). A functional equivalent of VEGF receptor molecule also includes modified VEGF receptor molecules that functionally resemble VEGF receptor molecules that can be used in the present invention (e.g., bind specifically to VEGF and possess low immunogenicity). For example, a functional equivalent of VEGF receptor molecule can contain a "SILENT" codon or one or more amino acid substitutions, deletions or additions. For example, a functional equivalent of VEGF receptor molecule can contain a substitution of one acidic amino acid for another acidic amino acid, or a substitution of one codon encoding the same or different hydrophobic amino acid for another codon encoding a hydrophobic amino acid. See Ausubel *et al., Current Protocols in Molecular Biology,* Greene Publishing Assoc. and Wiley-Interscience, New York (1989).

Techniques described herein for producing TNFα receptor molecules can be employed in producing VEGF receptor molecules that can be used in the present invention.

### Methotrexate

Presently available oral and intravenous formulations of methotrexate include Rheumatrex® methotrexate dose pack (Lederle Laboratories, Wayne, NJ); methotrexate tablets (Mylan Pharmaceuticals Inc., Morgantown, WV; Roxane Laboratories, Inc., Columbus, OH); and methotrexate sodium tablets, for injection and injection (Immunex Corporation, Seattle, WA) and methotrexate LPF® sodium (methotrexate sodium injection) (Immunex Corporation, Seattle, WA). Methotrexate is also available from Pharmacochemie (Netherlands). Methotrexate prodrugs, homologs and/or analogs (e.g., folate antagonists) can also be used in the present invention. Alternatively, other immunosuppressive agents (or drugs that suppress the immune system) can be used in the present invention.

### Administration

TNFα antagonists, VEGF antagonists, methotrexate and compositions of the present invention can be administered to an individual in a variety of ways. The routes of administration include intradermal, transdermal (e.g., in slow release polymers), intramuscular, intraperitoneal, intravenous (including infusion and/or bolus injection), subcutaneous, oral, topical, epidural, buccal, rectal, vaginal and intranasal routes. Other suitable routes of administration can also be used, for example, to achieve absorption through epithelial or mucocutaneous linings. TNFα antagonists, VEGF antagonists, and compositions of the present invention can also be administered by gene therapy wherein a DNA molecule encoding a particular therapeutic protein or peptide is administered to the patient, e.g., via a vector, which causes the particular protein or peptide to be expressed and secreted at therapeutic levels *in vivo.* In addition, TNFα antagonists, VEGF antagonists, methotrexate and compositions of the present invention can be administered together with other components of biologically active agents, such as pharmaceutically acceptable surfactants (e.g., glycerides), excipients (e.g., lactose), carriers, diluents and vehicles. If desired, certain sweetening, flavoring and/or coloring agents can also be added.

TNFα antagonists, VEGF antagonists, methotrexate and compositions of the present invention can be administered prophylactically or therapeutically to an individual. TNFα antagonists can be administered prior to, simultaneously with (in the same or different compositions) or sequentially with the administration of a VEGF antagonist. TNF antagonists and VEGF antagonists can also be administered prior to, simultaneously with (in the same or different compositions) or sequentially with the administration of methotrexate. For example, TNF antagonists and VEGF antagonists can be administered as adjunctive and/or concomitant therapy to methotrexate therapy.

For parenteral (e.g., intravenous, subcutaneous, intramuscular) administration, TNFα antagonists, VEGF antagonists, methotrexate and compositions of the present invention can be formulated as a solution, suspension, emulsion or lyophilized powder in association with a pharmaceutically acceptable parenteral vehicle. Examples of such vehicles are water, saline, Ringer's solution, dextrose solution, and 5% human serum albumin. Liposomes and nonaqueous vehicles such as fixed oils can also be used. The vehicle or lyophilized powder can contain additives that maintain isotonicity (e.g., sodium chloride, mannitol) and chemical stability (e.g., buffers and preservatives). The formulation is sterilized by commonly used techniques.

Suitable pharmaceutical carriers are described in Gennaro, A.R., Ed., Remington's Pharmaceutical Sciences, 18th edition, Mack Publishing Co., Easton, PA (1990).

For example, a parenteral composition suitable for administration by injection is prepared by dissolving 1.5% by weight of active ingredient in 0.9% sodium chloride solution.

TNFα antagonists, VEGF antagonists and methotrexate are administered in therapeutically effective or synergistic amounts; compositions of the present invention are administered in a therapeutically effective or synergistic amount. As used herein, a therapeutically effective amount is such that co-administration of TNFα antagonist and VEGF antagonist, or administration of a composition of the present invention, results in inhibition of the biological activity of TNFα and VEGF relative to the biological activity of TNFα and VEGF when therapeutically effective amounts of TNFα antagonist and VEGF antagonist are not co-administered, or relative to the biological activity of TNFα and VEGF when a therapeutically effective amount of the composition is not administered. A therapeutically effective amount is also an amount such that co-administration of TNFα antagonist, VEGF antagonist and methotrexate results in inhibition of the biological activity of TNFα and VEGF relative to the biological activity of TNFα and VEGF when therapeutically effective amounts of TNFα antagonist, VEGF antagonist and methotrexate are not co-administered. A therapeutically effective amount is that amount of TNFα antagonist and VEGF antagonist necessary to synergistically or significantly reduce or eliminate symptoms associated with a particular TNF-mediated disease. A therapeutically effective amount is also that amount of TNFα antagonist, VEGF antagonist and methotrexate necessary to synergistically or significantly reduce or eliminate symptoms associated with a particular TNF-mediated disease. As used herein, a therapeutically effective amount is not an amount such that administration of TNFα antagonist alone, administration of VEGF antagonist alone, or administration of methotrexate alone, must necessarily result in inhibition of the biological activity of TNFα or VEGF.

Once a therapeutically effective or synergistic amount has been administered, a maintenance amount of TNFα antagonist alone, of VEGF antagonist alone, of methotrexate alone, or of a combination thereof, can be administered to the individual. A maintenance amount is the amount of TNFα antagonist, VEGF antagonist, methotrexate, or combination thereof, necessary to maintain the reduction or elimination of symptoms achieved by the therapeutically effective dose. The maintenance amount can be administered in the form of a single dose, or a series or doses separated by intervals of days or weeks.

The dosage administered to an individual will vary depending upon a variety of factors, including the pharmacodynamic characteristics of the particular therapeutic agent, and its mode and route of administration; size, age, health, sex, body weight and diet of the recipient; nature and extent of symptoms of the disease being treated, kind of concurrent treatment, frequency of treatment, and the effect desired. *In vitro* and *in vivo* methods of determining the inhibition of TNFα are well known to those of skill in the art. Such *in vitro* assays can include a TNF cytotoxicity assay (e.g., the WEHI assay or a radioimmunoassay, ELISA). *In vivo* methods can include rodent lethality assays, primate pathology model systems (see, e.g., Mathison *et al., J. Clin. Invest., 81*: 1925-1937 (1988); Beutler *et al., Science 229:* 869-871 (1985); Tracey et *al., Nature, 330:* 662-664 (1987); Shimamoto *et al., Immunol. Lett., 17*: 311-318 (1988); Silva *et al., J. Infect. Dis., 162:* 421-427 (1990); Opal *et al., J. Infect. Dis., 161:* 1148-1152 (1990); and Hinshaw *et al., Circ. Shock, 30*:279-292 (1990)) and/or rodent models of arthritis (Williams *et al., Proc. Natl. Acad. Sci. USA, 89*:9784-9788 (1992)). In patients with rheumatoid arthritis, TNFα blockade can be monitored by monitoring IL-6 and C-reactive protein levels (Elliott *et al., Arth. Rheum., 36*:1681-1690 (1993)). Methods of determining inhibition of VEGF are also well known to those of skill in the art (e.g., ELISA).

TNFα antagonists, VEGF antagonists and methotrexate can be administered in single or multiple doses depending upon factors such as nature and extent of symptoms, kind of concurrent treatment and the effect desired. Thus, other therapeutic regimens or agents (e.g., multiple drug regimens) can be used in combination with the therapeutic administration of TNFα antagonists, VEGF antagonists and methotrexate. Adjustment and manipulation of established dosage ranges are well within the ability of those skilled in the art.

Usually a daily dosage of active ingredient can be about 0.01 to 100 milligrams per kilogram of body weight. Ordinarily 1 to 40 milligrams per kilogram per day given in divided doses 1 to 6 times a day or in sustained release form is effective to obtain desired results. Second or subsequent administrations can be administered at a dosage which is the same, less than or greater than the initial or previous dose administered to the individual.

A second or subsequent administration is preferably during or immediately prior to relapse or a flare-up of the disease or symptoms of the disease. For example, the second and subsequent administrations can be given between about one day to 30 weeks from the previous administration. Two, three, four or more total administrations can be delivered to the individual, as needed.

Dosage forms (composition) suitable for internal administration generally contain from about 0.1 milligram to about 500 milligrams of active ingredient per unit. In these pharmaceutical compositions the active ingredient will ordinarily be present in an amount of about 0.5-95% by weight based on the total weight of the composition.

The present invention will now be illustrated by the following Example, which is not intended to be limiting in any way.

### EXAMPLES

### EXAMPLE 1 Treatment of RA patients Using Anti-TNFα Antibody

### METHODS

### Patients

Seventy-three (73) patients with active rheumatoid arthritis (RA) were enrolled in a multi-centre, randomised, placebo-controlled double-blind clinical trial of anti-TNFα antibody. All patients met the criteria of the American College of Rheumatology (active RA for ≥ 6 months, failed treatment with at least one disease-modifying drug and evidence of erosive disease on radiography of hands and feet) (Arnett *et al.*, *Arth. Rheum., 31*:315-324 (1988)). In addition, all patients had previously received an average of 3 disease-modifying drugs, treatment with which was withdrawn at least 4 weeks prior to randomisation and entry to the trial (Elliott *et al., Lancet*:1105-1110 (1994)). Active disease was defined by the presence of six or more swollen joints plus at least three of four secondary criteria (duration of morning stiffness ≥ 45 minutes; ≥ 6 tender or painful joints; erythrocyte sedimentation rate (ESR) ≥ 28 mm/h; C-reactive protein (CRP) ≥ 20 mg/l (Elliott M.J. *et al., Lancet, 344*:1105-1110 (1994))

### Study Infusions

Chimeric monoclonal anti-TNFα antibody cA2 was supplied as a sterile solution containing 5 mg cA2 per ml of 0.01 M phosphate-buffered saline in 0.15 M sodium chloride with 0.01% polysorbate 80, pH 7.2 (Centocor, Inc., Malvern, PA). The placebo vials contained 0.1% human serum albumin in the same buffer. Before use, the appropriate amount of cA2 or placebo was diluted to 300 ml in sterile saline by the pharmacist, and administered intravenously via a 0.2 µm in-line filter over 2 hours. The characteristics of the placebo and cA2 infusion bags were identical, and the investigators and patients did not know which infusion was being administered.

### Treatment Protocol and Serum Samples

Patients were randomized to a single infusion of placebo, 1 mg/kg or 10 mg/kg anti-TNFα monoclonal antibody cA2. Serum samples were available from 69 of the 73 patients. Serum samples were obtained before and up to 4 weeks after infusion of placebo (n=24) or cA2 at either 1 mg/kg (n=24) or 10 mg/kg (n=21) and compared with 53 normal individuals.

### Preparation of Cells

Synovial membrane samples, obtained from RA patients undergoing total joint replacement were digested with 5 mg/ml type IV colagenase (Sigma, UK) and 150 µg/ml type I DNAse (Sigma, UK) (Brennan F.M. *et al., Lancet 2*:244-247 (1989)). Tissue was pipetted through a 200 µl nylon mesh, and cells were cultured in 60mm² wells in RPMI plus 5% foetal calf serum (FCS; BioWhittaker, Belgium) in the absence or presence of 10 µg/ml anti-TNFα monoclonal antibody cA2, alone or together with 10 µg/ml IL-1 receptor antagonist (IL-1ra, from Dr. A. Berger, Upjohn Laboratories, MI). Synovial fibroblasts were selected by continuously culturing synovial membrane cells until a confluent monolayer of fibroblasts was obtained. Adherent cells were further passaged using 0.05% trypsin/0.02% EDTA and cultured in RPMI and 10% FCS. The human dermal microvascular endothelial cell line HMEC-1 was a gift from the Center for Disease Control and Prevention (Atlanta, GA), and the human monocytic cell line THP-1 was from the American Tissue Culture Collection (Rockville, MD). Adherent cells (endothelial cells and fibroblasts) were cultured at confluent density in 200 mm² wells. THP-1 cells were suspended at a density of 0.5 x 10⁶/60 mm² well. Cells were stimulated for 72 hours in RPMI plus 5% FCS, in the absence or presence of either TNFα (gift from Prof. W. Stec, Centre of Molecular and Macromolecular Studies, Lodz, Poland) or IL-1α (gift from Hoffmann La Roche, USA).

### VEGF Assays and Statistical Analyses

VEGF in culture supernatants and serum samples was assayed by enzyme-linked immunosorbent assay (ELISA) (R&D Systems, UK). Patient data were analyzed by Wilcoxon signed rank test for comparisons within groups, using individual data as input variables, and between treatment groups by Mann-Whitney U-test, using % change from week 0 as response variables. Differences in VEGF release by RA synovial membrane cells between treatment groups were assessed by Mann-Whitney U-test.
Comparisons between multiple groups were adjusted using the Bonferroni correction.

### ESR and CRP Assessments

ESR was measured with a standard method (Westergen). CRP levels were measured by rate nephelometry (Abbott fluorescent polarizing immunoassay). See also, Elliott *et al., Lancet 344*:1105-1110 (1994); Elliott *et al., Lancet 344*:1125-1127 (1994); and Elliott *et al., Arthritis Rheum. 36*(12):1681-1690 (1993), which references are entirely incorporated herein by reference.

### RESULTS

### Serum VEGF Levels Are Elevated In RA Patients: Effect Of Treatment With Anti-TNFα Antibody.

Serum VEGF concentrations were measured by ELISA in a total of 53 age- and sex-matched non-arthritic individuals and 69 patients with active RA. To assess the degree of correlation between CRP and VEGF, the Kendall rank correlation coefficient for non-parametric data was calculated.

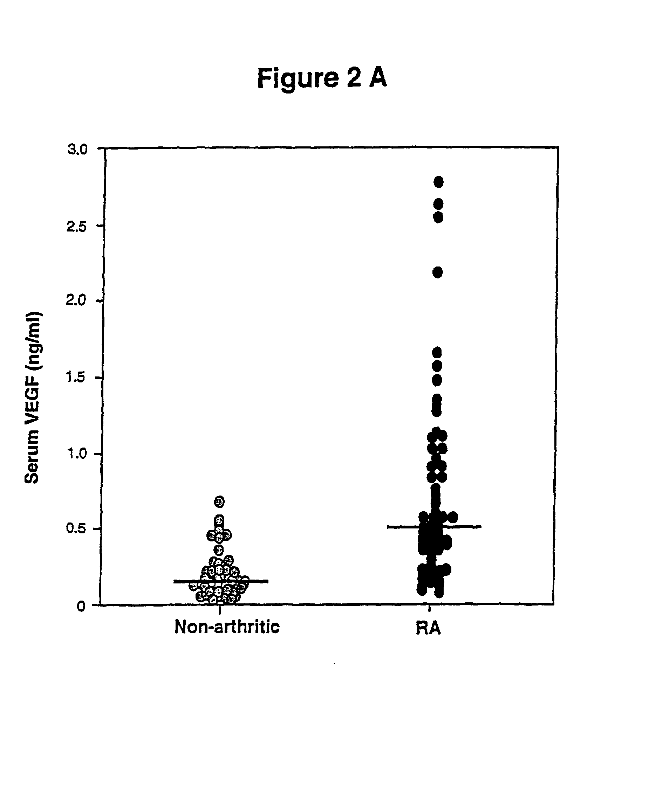

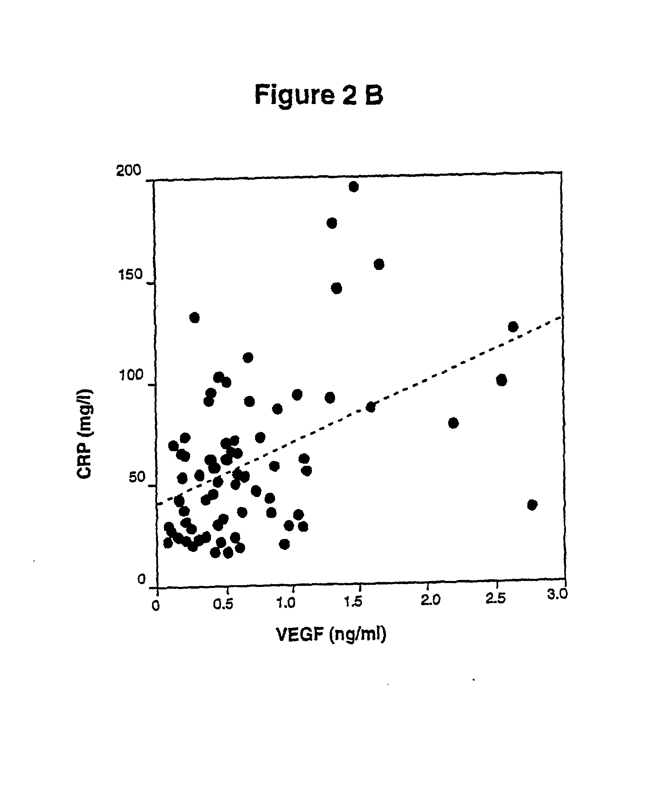

Median serum VEGF levels in the 53 non-arthritic individuals were equivalent to 160 pg/ml (interquartile range 122-266 pg/ml). In contrast, serum VEGF concentrations in the 69 patients with active RA were markedly elevated (median 503 pg/ml, range 307-887 pg/ml, p<0.001 versus non-RA; Figure 2A), and correlated with circulating CRP values (Kendall rank correlation co-efficient 0.252, p<0.01; Figure 2B), but not with individual clinical parameters of disease, such as the number of swollen joints or early morning stiffness.

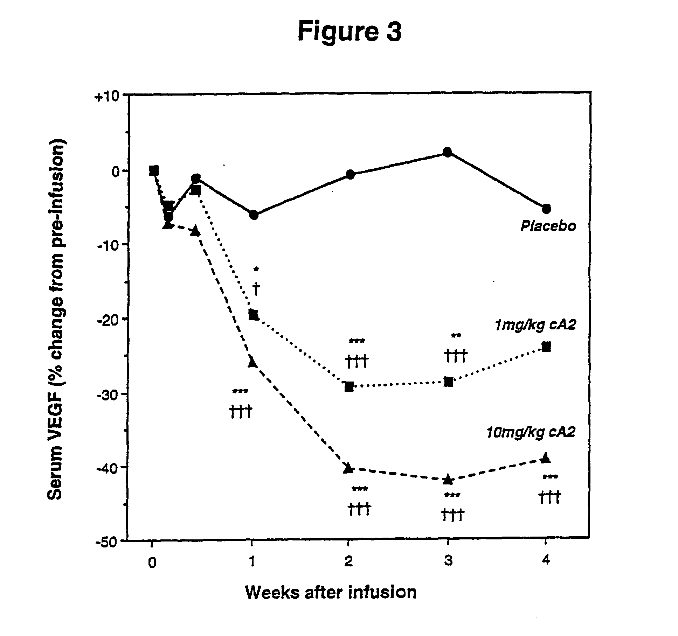

Treatment of RA patients with anti-TNFα significantly reduced serum VEGF (Figure 3). Values were expressed as change from pre-infusion for each patient prior to calculation of % median change for each treatment group. Data were analysed using the Wilcoxon signed rank test for comparisons within groups (* p≤0.05, **p≤0.01, ***p≤0.001), and between treatment groups by Mann-Whitney U-test (+ p≤0.05, +++ p≤0.001). Significance values for comparisons between multiple groups were adjusted using the Bonferroni correction.

In patients receiving 1 mg/kg cA2, the earliest change in VEGF concentrations was observed one week after infusion, and the maximal decrease (30%, p<0.001 versus pre-infusion and versus change in placebo) was attained at week two, after which serum VEGF concentrations returned to pre-treatment values. In patients who received 10 mg/kg cA2, the maximal change in serum VEGF concentrations was achieved at week 3 (decrease 42%, p<0,001 versus pre-infusion and versus change in placebo), and even 4 weeks after anti-TNFα serum VEGF concentrations were significantly below pre-infusion values (Figure 3).

### VEGF Secretion By Synovial Membrane Cells Is Dependent On Pro-inflammatory Cytokines.

To determine whether VEGF expression in RA is directly dependent on TNFα and IL-1, dissociated RA synovial membrane cells (1 x 10⁶ cells/ml; from RA joints) were cultured for two days in the absence or presence of inhibitors of cytokine bio-activity (10 µg/ml cA2, either alone or in combination with 10 µg/ml IL-lra). VEGF concentrations in culture supernatants were determined by ELISA. Statistical analyses *versus* VEGF release in the absence of cytokine inhibitors were performed by Mann-Whitney U-test. Results are shown in Table 1. Values in the Table are ng/ml VEGF, and are representative of 5-6 experiments, with 1-6 determinations per experiment.

**Table 1**

| Addition | Median | Interquartile range | Significance |
|---|---|---|---|
| None | 3.29 | 2.03-6.96 | |
| Anti-TNFα | 2.56 | 1.47-6.46 | NS |
| None | 3.95 | 1.48-7.98 | |
| Anti-TNFα + IL-1 RA | 1.91 | 0.74-3.97 | p<0.05 |

Synovial membrane cells were found to release VEGF spontaneously in a total of 29/32 experiments, with immunoreactive protein detected in culture supernatants approximately 12 hours after isolation. In the presence of 10 µg/ml anti-TNFα antibody cA2, median release of VEGF on day 2 in culture was decreased by 22%, from a median value of 3.29 ng/ml to 2.56 ng/ml (mean of 6 experiments), although this reduction was not statistically significant (see Table). However, in a second set of experiments, addition of a combination of cA2 (10 µg/ml) and IL-1ra (10 µg/ml) markedly reduced production of VEGF from 3.95 ng/ml to 1.91 ng/ml (mean of 5 experiments; inhibition 52%, p<0.05 versus release from untreated cells; see Table). Similarly, 5 days after plating VEGF release was reduced by cA2 plus IL-1ra from 13.3 ng/ml to 9.5 ng/ml (mean inhibition 29%, p<0.01 versus release from untreated cells; mean of 2 experiments).

### TNFα Induces VEGF Release From Monocytic And Endothelial Cell, But Not FromSynovial Fibroblasts.

The cytokine dependence of VEGF production from cells known to be able to express VEGF in RA joints (Koch *et al., J. Immunol., 152*:4149-4156 (1994): and Fava *et al., J. Exp. Med., 180*:341-346 (1994)) was also investigated. Monocytic cells (THP-1), human microvascular endothelial cells (HMEC-1) and human RA synovial membrane fibroblasts were incubated in the absence or presence of 10 ng/ml TNFα or IL-1α for 72 hours. VEGF release into the supernatants was measured by ELISA.

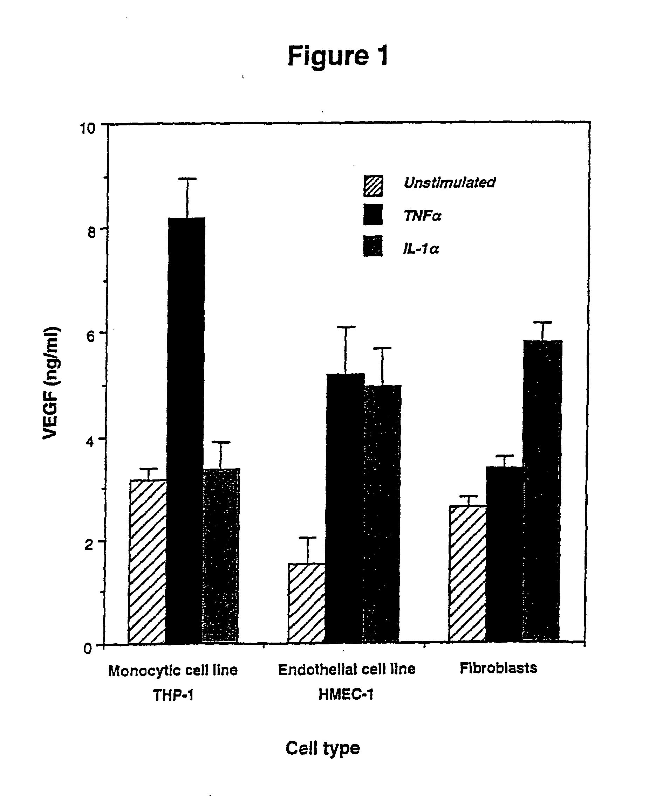

RA synovial membrane fibroblasts and monocytic THP-1 released VEGF spontaneously (Figure 1). In addition, microvascular endothelial cells were also found to constitutively release significant amounts of VEGF protein (Figure 1). However, although VEGF secretion from THP-1 monocytic cells was markedly increased by TNFα (fold increase equivalent to 2.61) but not by IL-1α, the response of RA synovial membrane fibroblasts to TNFα was lower (fold increase 1.29) than that induced by IL-1α (fold increase 2.23; Figure 1). In contrast, endothelial cells were almost equally responsive to TNFα and IL-1α (fold increase 3.37 and 3.22, respectively; Figure 1). These data suggest that VEGF release from cell types representative of those present in RA joints can be induced by pro-inflammatory cytokines, and that TNFα and IL-1 differentially modulate VEGF release from different cell subtypes.

### EXAMPLE 2 Treatment of RA patients Using Anti-TNFα Antibody In Combination With Methotrexate

### METHODS

### Patients

Forty-three (43) patients who had been using methotrexate for at least 6 months, had been on a stable dose of 7.5 mg/week methotrexate for 4 weeks, and had active RA, were enrolled in a multi-centre, randomised, placebo-controlled double-blind clinical trial of anti-TNFα antibody in adjunct to methotrexate treatment. All patients met the criteria of the American College of Rheumatology (active RA for ≥ 6 months, failed treatment with at least one disease-modifying drug and evidence of erosive disease on radiography of hands and feet) (Arnett *et al., Arth. Rheum., 31*:315-324 (1988)). Active disease was defined by the presence of six or more swollen joints plus at least three of four secondary criteria (duration of morning stiffness ≥ 45 minutes; ≥ 6 tender or painful joints; erythrocyte sedimentation rate (ESR) ≥ 28 mm/h; C-reactive protein (CRP) ≥ 20 mg/l (Elliott M.J. *et al., Lancet, 344*:1105-1110 (1994)).

### Study Infusions

Chimeric monoclonal anti-TNFα antibody cA2 was supplied as a sterile solution containing 5 mg cA2 per ml of 0.01 M phosphate-buffered saline in 0.15 M sodium chloride with 0.01% polysorbate 80, pH 7.2 (Centocor, Inc., Malvern, PA). The placebo vials contained 0.1% human serum albumin in the same buffer. Before use, the appropriate amount of cA2 or placebo was diluted to 300 ml in sterile saline by the pharmacist, and administered intravenously via a 0.2 µm in-line filter over 2 hours. The characteristics of the placebo and cA2 infusion bags were identical, and the investigators and patients did not know which infusion was being administered.

### Treatment Protocol and Serum Samples

Patients were randomized to one of seven treatment groups. The number of patients in each dose (or treatment) group is indicated in Table 2. Each of the 43 patients received multiple infusions of either 0, 1, 3 or 10 mg/kg cA2. Infusions were at weeks 0, 2, 6, 10 and 14. Starting at week 0, the patients were receiving 7.5 mg/week methotrexate (Pharmacochemie, Netherlands) or 3 placebo tablets/week (Pharmacochemie, Netherlands). Patients were monitored for adverse events during infusions and regularly thereafter, by interviews, physical examination and laboratory testing. Serum samples were obtained before and up to 28 weeks after the initial infusion.

VEGF assays, ESR and CRP assessments, and statistical analyses were preformed as described in Example 1.

**Table 2**

| cA2 (mg/kg) | MTX (7.5 mg/week) | Patients Evaluated |
|---|---|---|
| 0 | + | 5 |
| 1 | + | 6 |
| | - | 7 |
| 3 | + | 6 |
| | - | 6 |
| 10 | + | 7 |
| | - | 6 |

### RESULTS

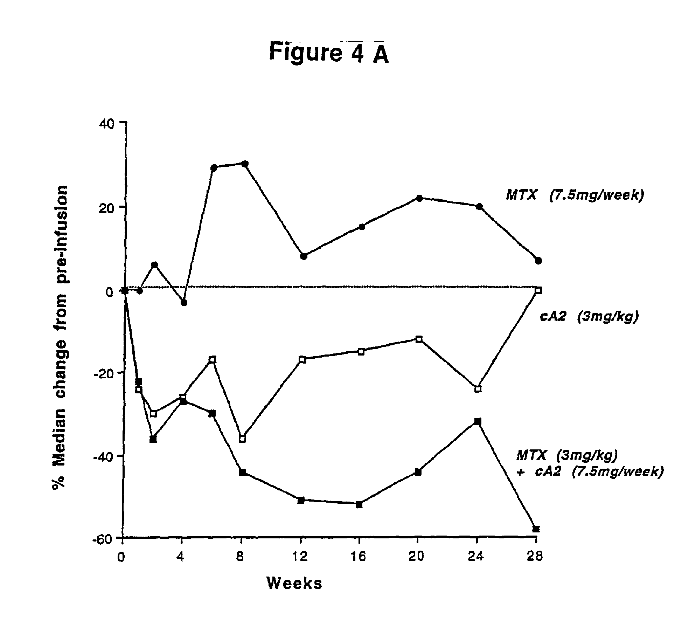

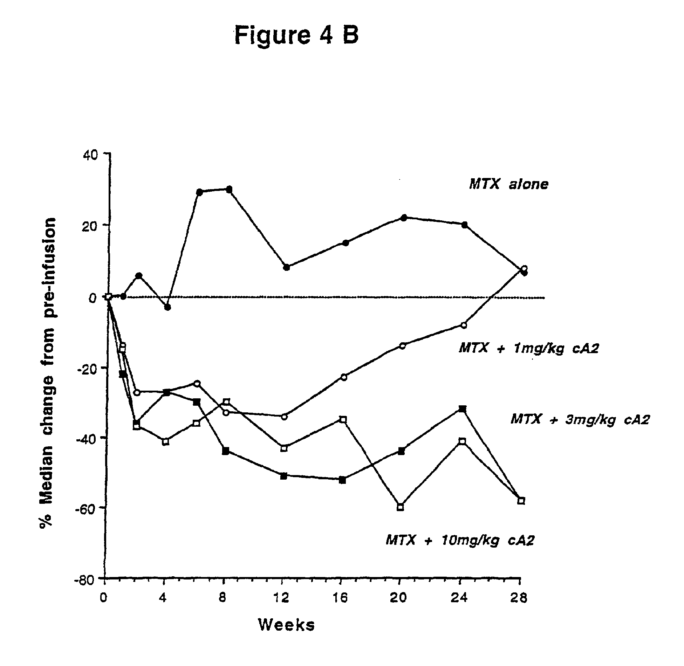

Treatment of RA patients with a combination of cA2 and methotrexate results in a more prolonged decrease in serum VEGF levels relative to patients who received either cA2 alone or methotrexate alone (Figures 4A and 4B). For example, although infusion of 3 mg/kg cA2 alone markedly reduced circulating VEGF levels, these returned to pre-infusion concentrations after the final infusion (serum VEGF levels at week 20 were equivalent to 87% of pre-infusion). In contrast, in patients who received cA2 in combination with methotrexate, the reduction in circulating VEGF levels was maintained even 6 weeks after the final infusion of cA2 (serum VEGF levels at week 20 were equivalent to 56% of pre-infusion; Figure 4A). This effect was dependent on the dose of cA2. That is, the reduction in serum VEGF levels was more sustained in patients who received 3 or 10 mg/kg cA2 in combination with methotrexate than in patients who received 1 mg/kg cA2 in combination with methotrexate (Figure 4B).

### Summary

The results in Examples 1 and 2 demonstrate that pro-inflammatory cytokines, including TNFα and IL-1, regulate the major mediator of angiogenesis, VEGF, during the pathogenesis of RA, and that blockade of cytokine activity may modulate new blood vessel formation. In particular, as judged by the decrease in serum concentrations after anti-TNFα antibody treatment, TNFα modulates production of VEGF *in vivo,* suggesting that part of the benefit of anti-TNFα antibody treatment may be due to reduction in angiogenesis, and that long term TNFα blockade can reduce neovascularisation and hence the cellular mass of the pannus and its destructive potential. VEGF is an appropriate therapeutic target in RA for achieving synergism with anti-TNFα therapy, leading to long term benefit.

### EQUIVALENTS

Those skilled in the art will be able to recognize, or be able to ascertain, using no more than routine experimentation, many equivalents to the specific embodiments of the invention described herein. Such equivalents are intended to be encompassed by the following claims.

## Claims

1. Use of a tumor necrosis factor alpha antagonist and a vascular endothelial growth factor antagonist for the manufacture of compositions for treating or preventing a tumor necrosis factor-mediated disease in an individual; the tumor necrosis factor-mediated disease e.g. being selected from the group consisting of: autoimmune disease, acute or chronic immune disease, inflammatory disease and neurodegenerative disease.

2. Use of claim 1, further comprising the co-use of methotrexate for the manufacture of the therapeutically or prophylactically effective compositions.

3. Use of claim 1 wherein the tumor necrosis factor alpha antagonist is effective to prevent or inhibit tumor necrosis factor alpha synthesis, tumor necrosis factor alpha release or its action on target cells, and e.g. the tumor necrosis factor alpha antagonist is an anti-tumor necrosis factor alpha antibody or antigen-binding fragment thereof, e.g. a chimeric antibody, such as (a) chimeric cA2 antibody or an antigen-binding fragment thereof, or (b) antibody or antigen-binding fragment thereof which competitively inhibits the binding of a chimeric cA2 antibody or an antigen-binding fragment thereof to human tumor necrosis factor alpha.

4. Use of claim 3 wherein the tumor necrosis factor alpha antagonist is (i) a receptor molecule that binds tumor necrosis factor alpha, and e.g. the receptor molecule is a tumor necrosis factor alpha receptor/immunoglobulin G fusion protein; or (ii) a phosphodiesterase inhibitor such as pentoxifylline; or (iii) thalidomide.

5. Use of claim 1 wherein the vascular endothelial growth factor antagonist is an anti-vascular endothelial growth factor antibody or an antigen-binding fragment thereof, and e.g. the tumor necrosis factor alpha antagonist is an anti-tumor necrosis factor alpha antibody or an antigen-binding fragment thereof.

6. Use of a tumor necrosis factor alpha antagonist and a vascular endothelial growth factor antagonist and optionally methotrexate in the manufacture of compositions treating or preventing rheumatoid arthritis in an individual in need thereof.

7. Use of a tumor necrosis factor alpha antagonist and a vascular endothelial growth factor antagonist and optionally methotrexate, for the manufacture of compositions for treating or preventing Crohn's disease in an individual in need thereof.

8. Use of a tumor necrosis factor alpha antagonist and a vascular endothelial growth factor antagonist, and optionally methotrexate, for the manufacture of compositions for treating or preventing acute or chronic immune disease associated with a transplantation in an individual in need thereof.

9. Use of claim 8 wherein the transplantation is selected from the group consisting of: renal transplantation, cardiac transplantation, bone marrow transplantation, liver transplantation, pancreatic transplantation, small intestine transplantation, skin transplantation and lung transplantation.

10. Use of claim 6, 7, 8 or 9 wherein the tumor necrosis factor alpha antagonist is an anti-tumor necrosis factor alpha antibody, e.g. a chimeric antibody, or an antigen-binding fragment thereof.

11. Use of claim 6, 7, 8 or 9 wherein the vascular endothelial growth factor antagonist is an anti-vascular endothelial growth factor antibody or an antigen-binding fragment thereof, and optionally the tumor necrosis factor alpha antagonist is an anti-tumor necrosis factor alpha antibody or an antigen-binding fragment thereof.

12. A composition comprising a tumor necrosis factor alpha antagonist and a vascular endothelial growth factor antagonist, and optionally further comprising methotrexate.

13. A composition of claim 12 wherein the tumor necrosis factor alpha antagonist is an anti-tumor necrosis factor alpha antibody, e.g. a chimeric antibody, or an antigen-binding fragment thereof, and/or the vascular endothelial growth factor antagonist is an anti-vascular endothelial growth factor antibody or an antigen-binding fragment thereof.

14. A composition according to claim 12 or claim 13, for treating or preventing a tumor necrosis factor-mediated disease, such as autoimmune disease, acute or chronic immune disease, e.g. associated with transplantation, inflammatory disease and neurodegenerative disease; or for treating or preventing rheumatoid arthritis.

15. Use of a tumor necrosis factor alpha antagonist and a vascular endothelial growth factor antagonist, for the manufacture of compositions for treating or preventing a tumor necrosis factor-mediated disease, such as autoimmune disease, acute or chronic immune disease, e.g. associated with transplantation, inflammatory disease, neurodegenerative disease; or for the manufacture of compositions for treating or preventing rheumatoid arthritis.
